Europäisches Patentamt

**(19)** European Patent Office

Office européen des brevets

**(11)** Publication number : **0 355 230 B1**

# **(12)** EUROPEAN PATENT SPECIFICATION

**(45)** Date of publication of patent specification :
**11.10.95 Bulletin 95/41**

**(51)** Int. Cl.⁶ : **G02C 7/06,** G02C 7/04,
G02B 5/18

**(21)** Application number : **88310565.2**

**(22)** Date of filing : **10.11.88**

**(54)** Method of producing a multifocal diffractive optical element.

**(30)** Priority : **26.08.88 US 237292**

**(43)** Date of publication of application :
**28.02.90 Bulletin 90/09**

**(45)** Publication of the grant of the patent :
**11.10.95 Bulletin 95/41**

**(84)** Designated Contracting States :
**AT BE CH DE ES FR GB GR IT LI LU NL SE**

**(56)** References cited :
EP-A- 351 471
EP-A- 0 109 753
US-A- 4 210 391
US-A- 4 338 005
US-A- 4 340 283

**(73)** Proprietor : **Cohen, Allen L., Dr.**
**10010 Walsham Court**
**Richmond Virginia 23233 (US)**

**(72)** Inventor : **Cohen, Allen L., Dr.**
**10010 Walsham Court**
**Richmond Virginia 23233 (US)**

**(74)** Representative : **Harding, Charles Thomas et
al**
**D. Young & Co.**
**21 New Fetter Lane**
**London EC4A 1DA (GB)**

## Description

The invention relates to a method of manufacturing a multifocal diffractive optical element.

A diffractive multifocal optical element comprising a phase zone plate of a Cohen lens design containing rotationally symmetrical, curved repetitive patterns designed to operate at a specific wavelength and which are also curved in $r^2$ space. In particular, the invention embraces a diffractive multifocal optical element comprising a phase zone plate of a Cohen lens design, i.e., containing rotationally symmetrical repetitive patterns designed to operate at a specific wavelength which are periodic in accordance with $\sqrt{n}$ spacing, which

(a) have curved profiles that are also curved in $r^2$ space,

(b) contain modulations within the pattern sufficient to introduce a phase shift in the optical path of about $\lambda/8$ or more, where $\lambda$ is the designed wavelength, and

(c) are correlated such that the light intensity at two focal points comprise, for each focal point, at least 20 percent of the incoming light at $\lambda$, and the ratio of the intensities at the two focal points is between about 0.5 and 2.00.

## Background To The Invention

This invention relates to an improvement in phase zone plate optics embracing contact and intraocular lenses. A "phase zone plate", as employed herein and in the claims, is a unitary optical region of a lens utilizing the combination of a zone plate and optical facets (such as in the form of echelettes) in the zones of the zone plate, and the combined facets in the zones diffract light to produce a specific wavefront which results in a specific intensity distribution of light at a variety of orders (e.g., $0^{th}$, $1^{st}$, etc.) of the zone plate. The orders constitute the foci of the zone plate. In a restrictive sense and also in the most utilitarian sense, the phase zone plate is designed for general lens applications where the distribution of light at effective intensities is dependent upon zone spacing for yellow light. Yellow light, as employed herein, is that portion of the visible spectrum at 530 - 570 manometers.

This invention relates *inter alias* to a method of making contact lenses. Contact lenses are classical vergence type lenses. They possess a concave corneal bowl (the posterior surface) that allows fitting to the eye and the outer surface (the anterior surface) is smooth and shaped to allow the eyelid to slide over the eye and to provide proper vergence of light (taking the lens material's refractive index into consideration) to a focal point accommodating to the eye. The majority of the commercial contact lenses are shaped such that the lenses are thinnest about the optical axis and the depth of the lenses gradually increases along a sloped radial length extending in the direction of the lens perimeter. Owing to the difference in depth extending from the optical axis, light passing through the optical axis has to pass through less of the lens material. Because light travels faster in air, the light passing through greater depths relative to light passing through lesser depths will be shifted, hence be retarded in time. See Fincham, et al., Optics, Published by Butterworths, London, $9^{th}$ edition, 1980, 1981, pages 72 - 75. Consequently, the shape of the lens is selected to accommodate this progressive retardation of the light so that the lightwaves emanating from the posterior surface are in synchronization in reaching a desired focal point.

This invention concerns contact lenses utilizing phase zone plate optics, such as phase zone plate bifocals and "tuned" Fresnel lenses making use of concentric annular zones. Such lenses generally follow the designs described, for example, by Allen L. Cohen in U.S. 4,210,391; 4,338,005; and 4,340,283 ("Cohen patents"). A Cohen lens design provides that the radii "$r_n$" of the annular and concentric zones are substantially proportional to $\sqrt{n}$ and that the zones are cut so as to direct light to more than one focal point.

Prior to the work of Cohen, the analyses provided by Fresnel and Wood demonstrated that every full-period zone of a phase zone plate comprises two half-period zones (odd and even zones) which are sufficiently out of phase to destructively interfere at the $1^{st}$ order focal point. They taught that by blocking an odd or even zone out or phase shifting the light from an odd to even zone, destructive interference is minimized. However, this did not result in a useful bifocal lens. Cohen found that by making the odd and even zones different and thereby directing light differently, the incident parallel light passing through the zones would be coordinated to form a diffraction limited element that directs the light to different focal points thereby providing a useful multifocal lens. The differences in the odd and even zones are accomplished by variations in thickness or refractive index in and between the zones.

The Cohen lens design with phase zone plate optics allows bifocal lens constructions which are exceptionally thin. Contact lenses may be designed with phase zone plate optics in order to achieve a bifocal or other multifocal effects. The specific chromatic properties of a phase zone plate may be incorporated in the design of a contact lens including a contact lens having multifocal properties. All phase zone plate optical elements which are designated bifocals are possessed inherently with the ability to focus light to more than two focal points. They are designated bifocals because the intensity levels of the light to any two orders, e.g., the 0th and 1st order focal points are adequate for bifocal applications. In that sense, every bifocal distributes light to a third, and possibly more, focus. The judgment of whether a lens is a bifocal or trifocal is not based on any strict rule. If the wearer of the lens does not find objectionable the presence of the third or more focuses, then the lens is probably adequate as a bifocal. See Klein and Ho, SPIE, August 1986, Table 2 and the comments about Table 2.

Other references mentioning or suggesting phase zone plate optics in regards to contact lenses are G. Forst, "Research into the Usability of Circular Grids as Aid to Vision," **Der Augenoptiker**, 1966 (12), pages 9-19; Ziegler, "Fabrication or Correction of Optical Lenses," as modified by Cohen, see column 4, lines 27-36 of Cohen, U.S. Patent No. 4,339,005, and column 5, line 63 to column 6, line 68, of Cohen, U.S. Patent No. 4,210,391; Freeman, U.S. 4,637,697; and Freeman, U.S. 4,642,112 (to the extent that holography embraces phase zone plate optics).

Bifocal contact lenses utilizing the above principles of phase zone plate optics are commercially available. Such lenses are believed to utilize parabolically-profiled, stepped annular facets each comprising a full-period zone where each zone has a depth of an optical path length of $\lambda/2$, providing a physical depth of $\lambda/2(\eta'-\eta)$. $\eta'$ and $\eta$ are the indices of refraction of the lens and the medium (e.g., lachryrnal layer) in which the lens is interacting and $\lambda$ is the design wavelength, in this case that of yellow light. This results in a bifocal contact lens where the 0th and 1st orders have an equal split of yellow light intensity at about 40.1%.

A full-period zone, for purposes of this invention, is defined as the smallest repetitive sequence of facets within a phase zone plate which are spaced substantially proportional to $\sqrt{n}$. Such spacing is characterized by the formula:

$$r_n \simeq \sqrt{2\,n\,d\,\lambda}$$

where d represents the 1st order focal length. A half-period zone, for the purposes of this invention, is characterized by the formula:

$$r_n \simeq \sqrt{n\,d\,\lambda}$$

where d represents the 1st order focal length.

If one were to alter the depth of the steps of this lens design there would result in vastly different concentrations of intensity of the incoming light to the focal points. For example, a phase zone plate with full-period spacing of the echelettes where the depth of the step of each echelette is $\lambda$, and the profile of the echelette is parabolic, using the wavelength of yellow light as the design wavelength, the resulting lens is essentially monofocal to the 1st order to the user regardless of the shape of the carrier lens overall. As the depth of this step is reduced, the flattening out of the echelettes results in different variations in depth over this spacing consequently effecting the differently shaped odd and even zones of Cohen. With a depth greater than $\lambda/2$, the intensity of light at the 1st order is significantly greater than at the 0th order because the thickness differences between the odd and even zones are high. It is only when the depth of the echelettes are $\lambda/2$ that the lens provides uniform light intensities at the 0th and 1st orders. Here the thickness differences between the odd and even zones are balanced. When the depth of the echelettes falls below $\lambda/2$, the thickness differences between the odd and even zones become minimized and this results in greater light intensity at the 0th order.

What is lacking in the art is the flexibility to vary the depth of the steps yet retain a desirable split of the intensity of the light to the 0th and 1st orders or any other multiple order combinations. It would be desirable to have the ability to generate multifocal, especially bifocal, lenses in which the depth of the echelette may be a variable factor in lens design, yet the lens designer has the capacity to make a lens which has a favourable split of light to the 0th and 1st orders or any other combination of orders.

In accordance with the invention there is provided a method of manufacturing a multifocal optical element including at least one zone plate containing annular concentric zones which are spaced substantially proportional to $\sqrt{n}$, n being an integer, said zones including a facet with a non-parabolic profile having a depth $D_o$, the method comprising steps of:

firstly determining a profile depth $D_o$ by:

- selecting a non-parabolic profile;
- selecting a design wavelength and zeroth and first order focal lengths;
- calculating intensities of light at said design wavelength at the zeroth and first order focal points as a function of profile depth, said intensities being determined by evaluating the Kirchhoff diffraction integral for said non-parabolic profile;
  
  selecting that profile depth to be depth $D_o$ at which the intensities of light are equal at the zeroth and first order focal points; and
  
  subsequently producing a multifocal optical element having the profile depth $D_o$ thus determined.

Thus the invention provides a lens design method which employs phase plate optics yet is freed of the constraints of facet structure to the parabolic profile. The lens design of this invention allows the lens manufacturer to make a lens, especially a contact lens, that utilizes a wide selection of echelette configurations yet allows the manufacturer to achieve a desired split of light to the near and far focal points. This flexibility in lens design frees the lens manufacturer from such constraints in lens design as echelette depth, traditional parabolic or flat profiles, and the like considerations.

The method of the invention can thus provide a diffractive multifocal optical element comprising a phase zone plate of a Cohen lens design containing rotationally symmetrical, curved repetitive patters designed to operate a specific wavelength and which are also curved in $r^2$ space. In particular, the invention can provide to a diffractive multifocal optical element comprising a phase zone plate containing rotationally symmetrical repetitive patterns (*i*.) designed to operate at a specific wavelength and (*ii*.) which

(a) are periodic in according with $\sqrt{n}$ spacing,

(b) have curved profiles that are also curved in $r^2$ space (it is pointed out that a parabolic curved profile would be linear and not curved in $r^2$ space),

(c) contain modulations within the pattern sufficient to introduce a phase shift in the optical path of about $\lambda/8$ or more, where $\lambda$ is the design wavelength, and (d) are correlated such that the light intensity at two focal points comprise, for each focal point, at least 20 percent of the incoming light at $\lambda$, and the ratio of the intensities at the two focal points is between about 0.5 and 2.00.

Thus the invention can provide a diffractive multifocal optical element comprising a phase zone plate of a Cohen lens design containing correlated rotationally symmetrical, curved repetitive patterns designed to operate at a specific wavelength, which curved repetitive patterns are curved as well in $r^2$ space and the variability in thickness or the refractive index of the repetitive patterns are correlated such that the light intensity at two focal points comprise, for each focal point, at least 20 percent of the incoming light at $\lambda$, and the ratio of the intensities at the two focal points is between about 0.5 and 2.00.

One example of a product of the method is an ophthalmic contact lens containing at least two phase zone plates within its optic zone, at least one of which embraces the features of the aforementioned optical elements.

Another example of a product of the method is an ophthalmic contact lens having within its optic zone, (1) a phase zone plate embracing the features of the aforementioned optical elements and (2) a pure refractive portion, preferably in the form of one or more channels.

The invention is described hereinafter, by way of example only, with reference to the accompanying drawings in which:

Figure 1 illustrates an incident plane wave striking a diffractive bifocal optical element illustrating the typical parabolic shaped echelettes, having the $\sqrt{n}$ spacing pattern of the Cohen lens design, whereupon it is transformed into two (2) emergent spherical waves directed to different foci, thus depicting the general principles of multifocal diffraction.

Figure 2 is a curve illustrating the echelette profile cut from a typical prior art diffractive bifocal optical element, such as in a lens according to Ziegler, *supra*. The axis labeled **d** represents the echelette thickness and the axis labeled **r** represents the radial distance along the echelette.

Figure 2a is the curve of Figure 2 shown in $r^2$ space.

Figure 3 is a cross-section of one embodiment of optical element in accordance with the invention, the facets of which are depicted graphically in Figure 4.

Figure 4 is a curve illustrating a facet profile of one embodiment of the invention. The axis labeled **d** represents facet thicknesses and the axis labeled **r** represents the radial distance along the facets.

Figure 4a is the curve of Figure 4 shown in $r^2$ space.

Figure 5 compares the graphical profiles of the full-period spaced echelette zone of the prior art possessing the conventional parabolic profile and the half-period spaced facet zones containing a multi-profile interrupted structure.

Figures 6 through 13 provide graphical depictions of cross-sectional views of a variety of facet arrangements for optical elements within the scope of this invention.

The invention finds its most favoured employment in the manufacture of contact lenses. Optical elements can be incorporated on the anterior or posterior portion, or both, of a contact lens. The optical elements may be provided on contact lenses by lathing or molding. The invention is favorably employed in multifocal (especially bifocal) intraocular lens.

The present invention can provide a diffraction bifocal optical element. It utilizes a circularly blazed diffraction grating to achieve its multifocal properties as taught by Cohen. The blazed grating allows for adjusting the split of light between two focal points by adjusting both the facet depth $D_o$ and the profile of the blazed facet itself. The invention utilizes novel profiles for the facets of the optical element. The novel profiles are non-parabolic and non-linear. The shape of the profile is adjusted to the depth of the facet thereby providing maximum flexibility in lens design.

This invention can provide a diffractive multifocal optical element comprising a phase zone plate of a Cohen lens design containing rotationally symmetrical, curved repetitive patterns designed to operate at a specific wavelength and which are also curved in $r^2$ space. This means that the curvature of the profile is neither linear nor parabolic, the only profile curvatures that have been contemplated by the prior art.

In further detail, this invention can provide a diffractive multifocal optical element comprising a phase zone plate containing rotationally symmetrical repetitive patterns (*i.*) designed to operate at a specific wavelength and (*ii.*) which

(a) are periodic in accordance with $\sqrt{n}$ spacing, thus replicating the Cohen lens design,

(b) have curved profiles that are also curved in $r^2$ space, thus establishing that the profiles are not linear or parabolic,

(c) contain modulations (characterized by variability in lens thickness or refractive index) within the pattern sufficient to introduce a phase shift in the optical path of about $\lambda/8$ or more, where $\lambda$ is the designed wavelength, and

(d) are correlated such that the light intensity at two focal points comprise, for each focal point, at least 20 percent of the incoming light at $\lambda$, and the ratio of the intensities at the two focal points is between about 0.50 and 2.00; preferably, the patterns are correlated such that the light intensity at two focal points comprise, for each focal point, at least 30 percent of the incoming light at $\lambda$ and the ratio of the intensities at the two focal points is between about 0.75 and 1.50; most preferably, the two focal points are at the $0^{th}$ and $1^{st}$ orders and the ratios of intensities is between about 0.8 to about 1.2.

The present invention can provide a diffraction bifocal optical element utilizing a circularly blazed diffraction grating to achieve its multifocal properties wherein the blazed grating allows for adjusting the split of light between two focal points by adjusting both the facet depth $D_o$ and the profile of the blazed facet itself, and the blazed facet provides an alternating inclination divided in accordance with $\sqrt{n}$ zone spacing.

The facet arrangements of the invention include dividing what in the prior art is considered a full-period ($\lambda$) spacing of the facets into alternating inclined half-period ($\lambda/2$) faceted zones that contain only one non-refractive cylindrical (or essentially cylindrical) surface for every two alternating half-period zones, and such two alternating half-period zones are interconnected by a smooth surfaced facet which effects a phase shift of the design wavelength light between the half-period zones. All of the non-refractive essentially cylindrical or cylindrical surfaces may be at any depth less than about, e.g., three design wavelengths deep, i.e., $<3\lambda$. The alternating zones of the invention provide control of the split of light between the focal points of the lens. By adjustment of the profiled inclinations of the alternating zone, it is possible to vary the intensity of light to the desired focal points.

The invention can provide a diffraction bifocal optical element superimposed on, etched into and/or embedded within a surface layer of a lens possessing the ability to independently converge light to at least two (preferably two) primary focal points in which the element comprises alternating inclined half-period ($\lambda/2$) faceted zones that contain only one non-refractive cylindrical (or essentially cylindrical) surface for every two alternating half-period zones, and such two alternating half-period zones are interconnected by a smooth surfaced facet which effects a phase shift of the design wavelength light between the half-period zones. All of the non-refractive essentially cylindrical or cylindrical surfaces are less than about, e.g., three design wavelengths deep, i.e., $<3\lambda$.

Thus the claimed invention provides a method by which non-parabolic zone profiles can be chosen for an effective diffraction bifocal element. The method is illustrated by the following:

1. A determination is made to either hold the light intensity values of both the $0^{th}$ and $1^{st}$ orders equal and change the depth $\mathbf{D_o}$ of the facet, e. g., hold the light intensity values of both the $0^{th}$ and $1^{st}$ orders at about 40%, or hold the value of $\mathbf{D_o}$ and alter the intensity values of the $0^{th}$ and $1^{st}$ orders.

2. Select a transmission profile for the facets different from the generally accepted parabolic profiles.

3. Plug the equation of the new profile into the Kirchhoff diffraction integral and the result is plotted for the intensity at $0^{th}$ order and $1^{st}$ order on a scale of 0 to 100% on one axis against the variation in depth of the facet, viz..$4\lambda/\eta-1$, etc.

4. Where the resultant curves cross one another defines a useful profile at the facet depth for the crossover point.

The Kirchoff diffraction integral, equations 1a and 1b, is useful for the consideration of a surface relief phase plate:

$$(1a) \qquad I_n = T_n \cdot T_n^* \text{ and}$$

$$(1b) \qquad T_n = \int_0^{2\lambda_o d} \exp\{i\, k\, (\eta - 1)\Delta(\rho)\}\exp\{-2\pi i\, u\, \rho\}d\rho$$

with $\Delta(\rho)$ $\equiv$ surface relief profile

$\rho$ $\equiv r^2$

$r$ $\equiv$ radial distance outward from the center of the bifocal phase plate lens

$i$ $\equiv \sqrt{-1}$

$k$ $\equiv 2\pi/\lambda_o$

$\eta$ $\equiv$ refractive index of the bifocal phase plate lens

$u$ $\equiv -1/(2\lambda_o f_n) = -n/(2\lambda_o d)$

The integration of equation (1b) is taken from 0 to $2\lambda_o d$ to include both an odd and an even zone, so that the integral covers two half-period zones. From equation (1b), the transmission profile of the phase plate is characterized -

$$\exp\{i\,\phi\}\equiv e^{i\phi} \qquad \text{transmission profile} \quad (2a)$$
$$\phi \equiv k\,(\eta - 1)\Delta(\rho) \qquad \text{phase retardation} \quad (2b)$$

In this process of generating a lens with a variable profile, a first step is to make a determination to either hold the light intensity values of both the $0^{th}$ and $1^{st}$ orders equal and change the depth $D_o$ of the facet, e. g., hold the light intensity values of both the $0^{th}$ and $1^{st}$ orders at about 40%, or hold the value of $D_o$ and alter the intensity values of the $0^{th}$ and $1^{st}$ orders. This process is followed by the selection of a transmission non-parabolic profile for plugging into the equations. An illustration of this is the following:

$$\Delta(\rho) = \Delta_o \cdot \{1/2 + 1/2 \cdot \cos(\pi\,\rho/r_1^2)\} \quad (3a)$$

In the above, $\Delta_o$ represents the maximum surface relief depth of the echelette. Plugging this into the Kirchoff diffraction integral, the results are

$$I_o = J_o^2(\zeta) \quad (3b)$$
$$I_1 = \{4[\sin(\zeta) - \zeta\cos(\zeta)]/(\pi\zeta^2) + J_2(\zeta)\}^2 \quad (3c)$$
$$I_{-1} = \{4[\sin(\zeta) - \zeta\cos(\zeta)]/(\pi\zeta^2) - J_2(\zeta)\}^2 \quad (3d)$$
$$\zeta \equiv 0.5k(\eta - 1)\Delta_o/\lambda_o$$

where $J_o$ and $J_2$ are bessel functions.

Plotting the results for the intensity at $0^{th}$ order and $1^{st}$ order on a scale of 0 to 100% on one axis against the variation in depth of the facet $\Delta_o$ demonstrates that one value of $\Delta_o$ equalizes the light intensity at the $0^{th}$ order and $1^{st}$ orders where the resultant curves cross one another. This value is

$$\Delta_o = 0.405\lambda_o/(\eta - 1)$$

This corresponds to a maximum phase retardation of

$$\phi_o = k\,(\eta - 1)\Delta_o = 0.81\,\pi\,\text{rad}$$

with light intensities at the $0^{th}$ and $1^{st}$ orders of about 40%.

A remarkable result of the invention is the minimal difference in inclination required in the alternating facets to achieve the benefits of excellent intensity of light at the designed focal points. For example, only a small difference in inclinations from a traditional parabolic shape is required in the half-period zones to generate a lens providing the advantages of the invention.

Such small differences come about by reason of the smallness of the facets even over a full-period zone measurement. For example, one embodiment of the invention may employ in a contact lens

◊ where the phase plate is characterized as comprising 8 full-period zones and is located in the posterior surface of the lens,

◊ the lens conforms to the shape of the eye and provides a typical refraction to the distant focal point, and

◊ the design wavelength is for yellow light, about 555 nanometers,

the following dimensions:

■ the first zone at the optical axis has a radius of about 0.75 millimeters;

■ the last zone away from the optical axis has a width defined by the difference in the radius to the outer periphery of the zone and the radius to the inner periphery of the zone, of about 0.14 millimeters; and

■ the depth of each facet is about 0.003 millimeters.

This same structure, measured however, in terms of its 16 half-period zone spacings caused by a profile surfaces inflection occurring at a point at about $\sqrt{n}$ dimensions:

■ the first zone at the optical axis has a radius of 0.053 millimeters;

■ the last zone away from the optical axis has a width defined by the difference in the radius to the outer periphery of the zone and the radius to the inner periphery of the zone, of about 0.067 millimeters.

On comparing the optical elements comprising facets that have a conventional parabolic shape over full-period zone spacing to optical elements comprising the half-period zone spacing and facet profiles of the invention, utilizing the depth of the step of 0.003 millimeter, the half-period zone spacing is found to have a slightly lower area under the curves reflecting the profile of the facets. That difference can be as little as about 1% area difference to about 10 % area difference. Typically the difference is about 2 to about 5% area difference. In the above illustration, the area difference is about 3%. As small as the area difference seems to be, its contribution to the performance of the lens is quite significant.

A lens having the prior art parabolic shaped echelettes, full-period spacing and with an echelette depth of 0.8 $\lambda/2$, provides the following light intensity distribution:

| -1 | 0 | 1 |
|----|----|----|
| .05 | .57 | .25 |

That is to be compared with the Klein and Ho description of the $\lambda/2$ analog showing the following intensities:

| m = | 3. nonalternating (b = .5) |
|----|----|
| -4 | .0050 |
| -3 | .0083 |
| -2 | .0162 |
| -1 | .0450 |
| 0 | .4053 |
| 1 | .4053 |
| 2 | .0450 |
| 3 | .0162 |
| 4 | .0083 |

A slight modification in the profile while maintaining the depth of 0.8 $\lambda/2$ yields a bifocal element encompassed by the invention that provides a light intensity distribution at 0th and 1st orders of .405.

The invention can provide a diffractive multifocal optical element comprising a phase zone plate containing annular concentric zones in which the zones are spaced substantially proportional to $\sqrt{n}$, the zones possess stepped facets that introduce a discontinuity in optical path length of less than $\lambda/2$. The invention also embraces a phase zone plate containing annular concentric zones possessing facets which provide an alternating stepped repetitive pattern in accordance with $\sqrt{n}$ spacing in the optical element and wherein the depth of the steps of the facets are less than $\lambda/2(\eta'-\eta)$, where $\eta'$ and $\eta$ are the indices of refraction of the lens and the medium in which the lens is interacting and $\lambda$ is the design wavelength.

In a particular embodiment of the invention, the optical element comprises facets within the annular concentric zones providing an alternating stepped repetitive pattern wherein:

1. the facet of one of the alternating zones has an inclined curved profile that is interrupted at the zone boundary by another curved profile providing the differently inclined curved facet of the other alternating zone,

2. the zones are spaced substantially proportional to $\sqrt{n}$,

3. the depth of the facets are less than $\lambda/2$,

4. the zones are cut so as to direct yellow light to at least two primary focal points in at least adequate intensities for visual usage at each such primary focal point, and,

5. but for the alternating pattern, the element would not have such intensity.

The invention can provide a bifocal optical element of the Cohen lens design wherein the odd and even zones of the phase zone plate

a. conform to $r_n \simeq \sqrt{n\,d\,\lambda}$ spacing,

b. are contiguous and free of a non-refractive step interface at at least every other zone boundary, and leave a sloped profile at such contiguous interface,

c. the cross-section of each odd zone has the same general profile and the cross-section of each even zone has the same general profile,

d. the general profile of the odd zones is different from that of the even zones, and

e. the depth of steps for the zones are less than $\lambda/2$.

Preferably, the slope profile provides a smooth transition from zone to zone.

In another aspect, the invention can provide a bifocal optical element of the Cohen design comprising a faceted step phase zone plate containing an alternating profile wherein:

a. the phase zone plate conforms to $r_n \simeq \sqrt{2\,n\,d\,\lambda}$ ;

b. the alternating profile occurs within the full-period spacing;

c. the facets have a depth less than about $\lambda/2$;

d. the zones are cut so as to direct yellow light to at least two primary focal points in at least adequate intensity for visual usage at each such primary focal point, and

e. but for such alternating profile the zones would not have such intensity for visual usage.

In a preferred embodiment, the optical element manufactured in accordance with the invention comprises optically diffractive facets providing two different curved profiles that are joined at radii $r_n$ through transition profiles located about such radii, which transition profiles have profile curvatures that are different from said two different curved profiles whereby to form annular and concentric zones at such transition profiles which zones are spaced substantially proportional to $\sqrt{n}$ and the zones are cut so as to direct yellow light to at least two primary focal points in at least adequate intensity for visual usage at each such primary focal point, which element but for the curved profiles would not have such intensity for visual usage.

This invention can provide an ophthalmic lens such as contact and intraocular lenses containing such optical elements. In a preferred embodiment of the invention, the ophthalmic lens is a bifocal lens that splits the light to two focal points in essentially equal intensities. In a most preferred embodiment of the invention, the optical element of the lens comprises a repetitive pattern of zones having a profile embraced by the equation

$$d = D_o \cdot \{1/2 + 1/2 \cdot \cos(\pi \cdot r^2/b^2)\}$$

where $d$ is the depth of the repetitive profile, $r$ is the radial position of the zone, $b$ is the radius of the 1st zone, and $D_o$ is the facet depth for the design wavelength.

Another benefit of the invention is the synergy and cooperative action existing between the alternating half-period zones of the invention to yield the excellent bifocal lens performance characteristics of the invention. For example, if either set of alternating zones were replicated for the other zones, thereby eliminating the alternation, the resulting lens would have a intensity distribution of parallel light and first order of the totally replicated odd zones of .85 and .075, respectively, and of the totally replicated even zones of .885 and .077, respectively. When they act together, they provide a far different intensity distribution.

In one embodiment of the invention, each facet of the alternating zones of the phase zone plate has a depth less than $\lambda/2$, where $\lambda$ is the design wavelength of the phase zone plate. In those cases where one zonal facet is joined by a curved profile to another zonal facet, if only one of them is formed from a step riser representing a non-refractive surface, in accordance with this embodiment, the two zonal facets will have a combined depth of less than $\lambda/2$. In this special case, and for convenience of calculations, the depth of the combination is viewed from the concept of full-period zone spacing. However, such alternating inclined zonal facets are viewed as having a variable depth. The depth of the facets may range from about 0.01 to about 0.99 times (x) $\lambda/2$, preferably about 0.05 to about .95 x $\lambda/2$, most preferably about 0.1 to about 0.9 x $\lambda/2$.

This invention makes it possible to construct a variety of facet (echelette) diffractive bifocal lenses with the requisite equal, or substantially equal, energy split between the two spherical waves going to the zeroth and first orders without being limited in facet depth.

This invention supports the novel concept that the energy split between the two emergent spherical waves is determined by a correlation of

1. facet (echelette) depths and

2. facet (echelette) profile.

It has been determined that by suitably contouring the facet (echelette) profiles an equal energy split is obtainable even when cutting a diffractive bifocal lens with facet (echelette) of any depth, assuming a reasonable limit of maximum depth of about three wavelengths, based on the design wavelength.

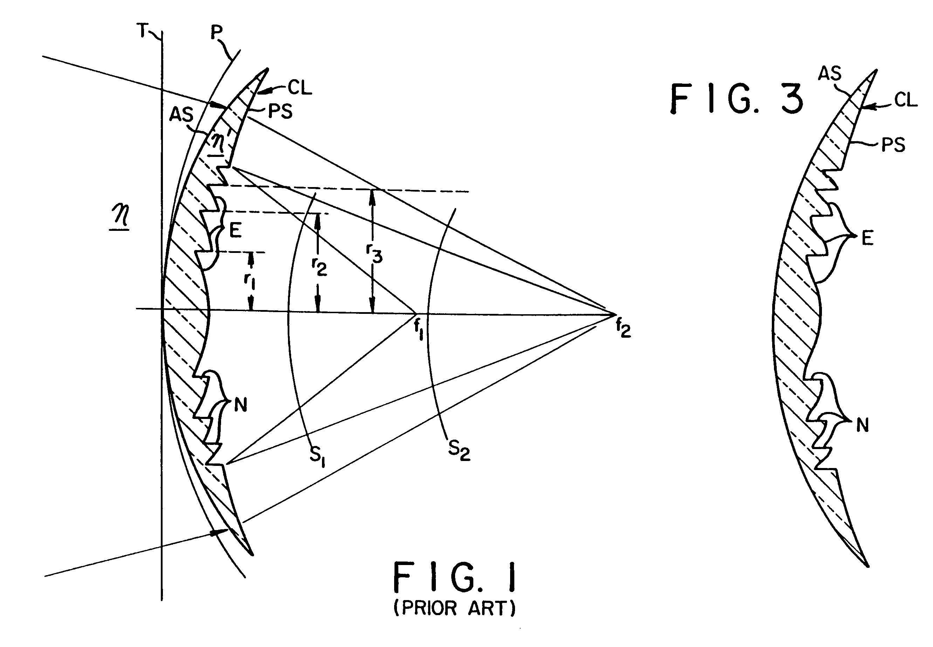

With respect to Figure 1, there is described a diffractive bifocal lens **CL** with a curvature to effect convergent refraction and diffraction. In the figure, optical elements **E** (predicated on a presumed full-period spacing) transform an incident plane wave into a wave front predominately of two spherical waves. For example, incident light wave with planar phase front **P** passes through the anterior surface **AS** of lens **CL** and emerges from the posterior surface **PS** as a light wave of predominately the two spherical phase fronts **S**$_1$ and **S**$_2$ with intensities **I**$_1$ and **I**$_2$, respectively. The posterior surface **PS** contains diffractive echelettes **E** and their corresponding non-optical edges **N**. The facet (echelette) spacing in a diffractive optical element is given by the standard formula

$$r_n \simeq \sqrt{n} \cdot r_1$$

in which **r**$_n$ is the radius of the n$^{th}$ zone (utilizing full-period spacing). And $\eta$ and $\eta'$ are the refractive indices of air and the lens **CL**, respectively. The location of the focal points of the two spherical wavefronts is determined by the radius of the first zone **r**$_1$ and the carrier power of lens **CL**. In particular, the m$^{th}$ order focal point **f**$_m$ is given by the equation

$$f_m = (r_1)^2/(2 \cdot \lambda \cdot m)$$

with $\lambda$ = wavelength; and m = 0, ±1, ±2, etc.

A desirable energy split has been suggested to occur when the two emergent spherical waves carry equal amounts of the total energy, that is, when **I**$_1$ = **I**$_2$. The current literature states that this is the case when the facet (echelette) depths **D**$_o$ are set at 1/2 wavelength deep (see the Klein and Ho, *supra*).

Figure 2 depicts a standard parabolic profile used in the prior art (see Ziegler, *supra*). The depth **d** of the repetitive profile as a function of radial position **r** is shown in the following equation:

$$d = D_o \cdot (1 - r^2/b^2) \qquad b = \text{radius of the 1}^{st}\text{ zone}$$

This profile is repeated in each zone, identical in **r**$^2$ space, but scaled down proportionally to the width of each such zone in the usual **r** space. The facet (echelette) depth for an equal energy split at the 0$^{th}$ and 1$^{st}$ orders is shown by the following equation:

$$D_o = 0.500 \cdot \lambda/(n - 1) \qquad n = \text{index of refraction}$$

and the intensity split is given by

$$I_1 = I_2 = (2.0/\pi)^2 = 0.405.$$

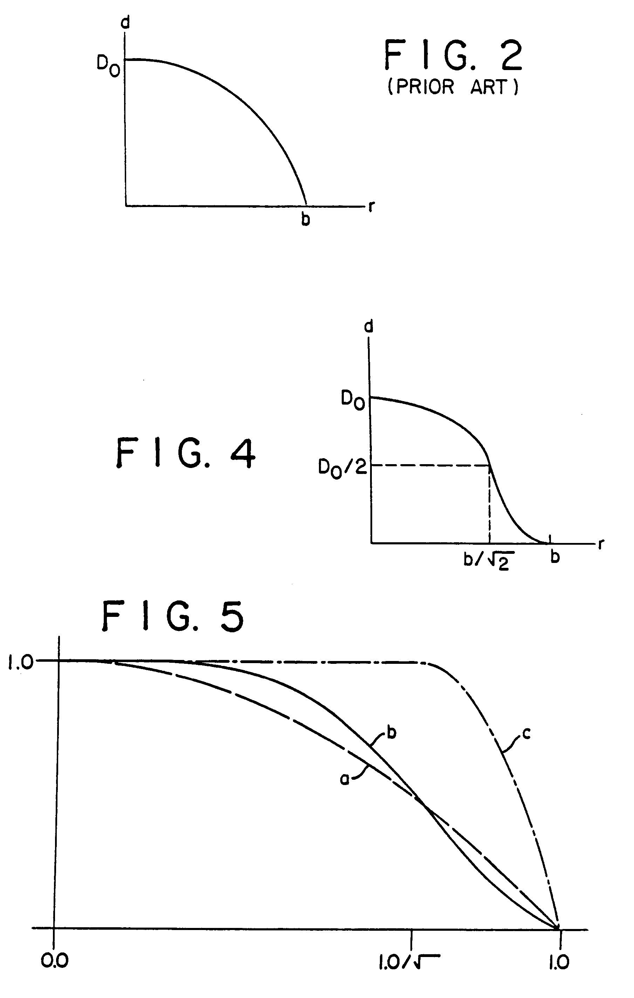

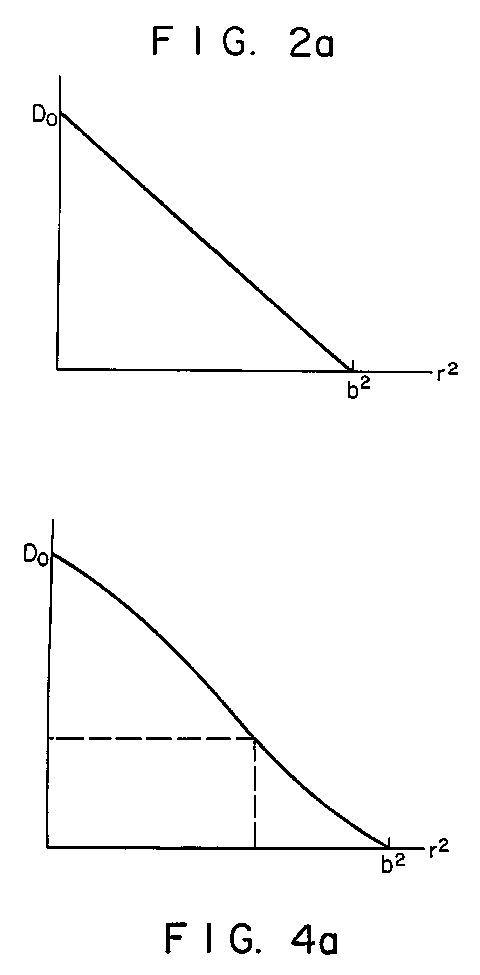

Figure 2a is the curve of Figure 2 shown in **r**$^2$ space demonstrating the difference between the profiles of the prior art and those of the invention, see Figure 4a in this respect.

Figure 3 shows a diffractive bifocal optical lens containing facets according to the design illustrated in Figure 4. The facet depths are .405/.500 = 80% of the depths required by prior art lenses.

Figure 4 illustrates a new cosine profile used in one embodiment of this invention. The repetitive profile is given by

$$d = D_0 \cdot \{1/2 + 1/2 \cdot \cos (\pi \cdot r^2/b^2)\}$$

The facet depth, utilizing a full-period spacing between the non-refractive edges of the steps but containing alternatingly inclined facets within half-period spacing, for an equal energy split is given by

$$D_o = 0.405 \cdot \lambda/(n - 1)$$

and the intensity split is given by

$$I_1 = I_2 = J_o^2(0.405 \cdot \pi) = 0.403$$

where **J**$_o$ is a bessel function.

Figure 4a illustrates the curve of Figure 4 in **r**$^2$ space demonstrating that the profiles of the phase plates of the invention retain some of their curvature even in **r**$^2$ space as contrasted with the phase plates of the prior art which lose all curvature in **r**$^2$ space, see Figure 2a in this respect.

Figure 3 comprises an optical lens **CL** possessing anterior surface AS and peripheral posterior surface **PS**. In this embodiment the posterior surface of the optic zone is comprised of the diffractive facets (echelettes) **E** and their corresponding non-optical edges **N**. The physical profile of the facets (echelettes) **E** is given by

$$d = D_o \cdot \{1/2 + 1/2 \cdot \cos (\pi \cdot r^2/b^2)\}$$

where **d** is the thickness of the facet (echelette), **r** is the radial distance from the inner edge of the zone (and such profile is repeated in each zone but scaled down proportionally to the width of each such zone) within which the facet (echelette) is formed and the occurrence of such profile alternation, and **b** is the radius of the first zone. This particular profile is drawn in Figure 4.

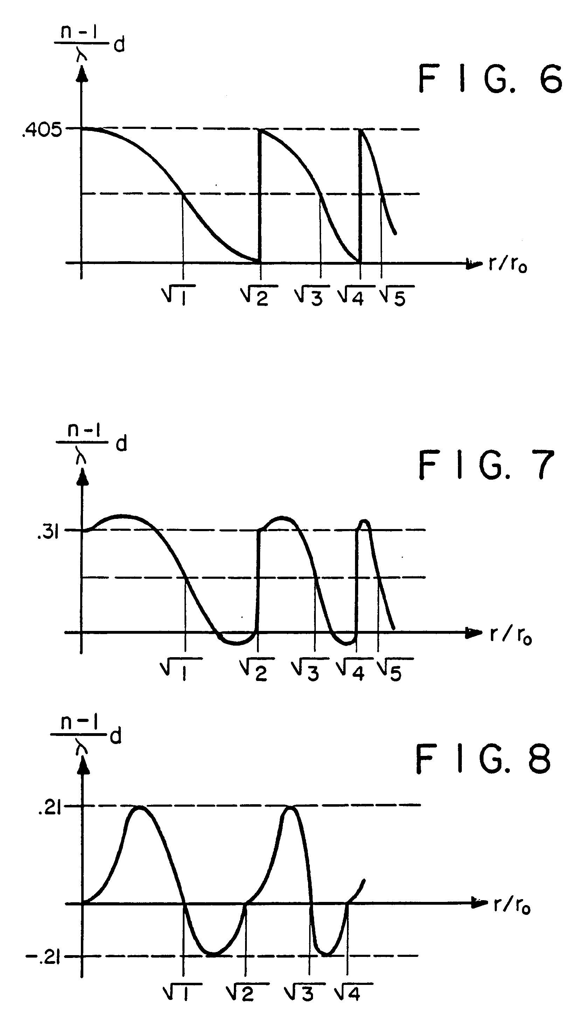

Figure 5 is an overlay of the parabolic echelette design **a** characteristic of the prior art (see Figure 2), the cosine profile **b** of Figure 4 and another useable profile **c** for a bifocal lens. The purpose of the overlay is to illustrate the profile differences between the structures of the invention and the prior art. Note particularly the shift in profile of curve **b** at the $\sqrt{n}$ spacing. That small difference allows the profile of curve **b** to be suitably employable as the facet profile for the lens element of the invention.

The physical profile of the facet **c** is given by

$$y = 1 - \{(r - 1/\sqrt{2})/(1 - 1/\sqrt{2})\}$$

Profiles **a** and **c** at such reduced depth fail to give an equal intensity split of the light to the $0^{th}$ and $1^{st}$ orders whereas they do when

$$D_o = 0.500 \cdot \lambda/(n - 1).$$

Figures 6 through 10 depict a variety of useful facet profiles that can be used according to the invention in the lens construction of Figure 2.

Figure 6 graphically depicts along an x-y axis the profile of Figure 4 in a repetitive sequence of alternating half-period inclined zones represented by the odd and the even zones. This particular embodiment is characterized by

$$y = 0.405\lambda/(n - 1) \cdot \{1/2 + 1/2 \cos (\pi r^2/2r_o^2\}$$

$$I_o = I_1 = 0.402$$

wherein $\lambda$ is the designed wavelength and $n$ is the index of refraction of the lens medium.

Figure 7 is another profile graphically depicted along an x-y axis in which the depth of the non-refractive edges of the step are further reduced to $0.31\lambda$ and the depth of the even half-period zones have a curved connection with the non-refractive edge. This embodiment of the invention is characterized by

$$y = 0.314\lambda/(n - 1) \cdot 2.5\{1/2 + 1/2 \cos (\pi r^2/2r_o^2\}$$

$$- 0.314\lambda/(n - 1) \cdot 1.5\{1 - r^2/2r_o^2\}$$

$$I_o = I_1 = 0.390$$

Figure 8 shows a profile where the edge of the step is inclined, suggesting that it contributes to the optical quality of the phase zone plate. The profiles of the half-period alternating zones in this embodiment are different from the preceding designs, mainly because the non-refractive edge has been substantially removed. This embodiment is characterized by

$$y = \lambda/(n - 1)\{r^2/r_o^2 + \cos(\lambda r^2/2r_o^2) - 1\}$$

$$I_o = I_1 = 0.314$$

It is to be noted in this embodiment that the depths of the facets for the odd zones were further reduced to $0.21\lambda$ but the even zones have a depth below the nadir of the odd zones by another $0.21\lambda$.

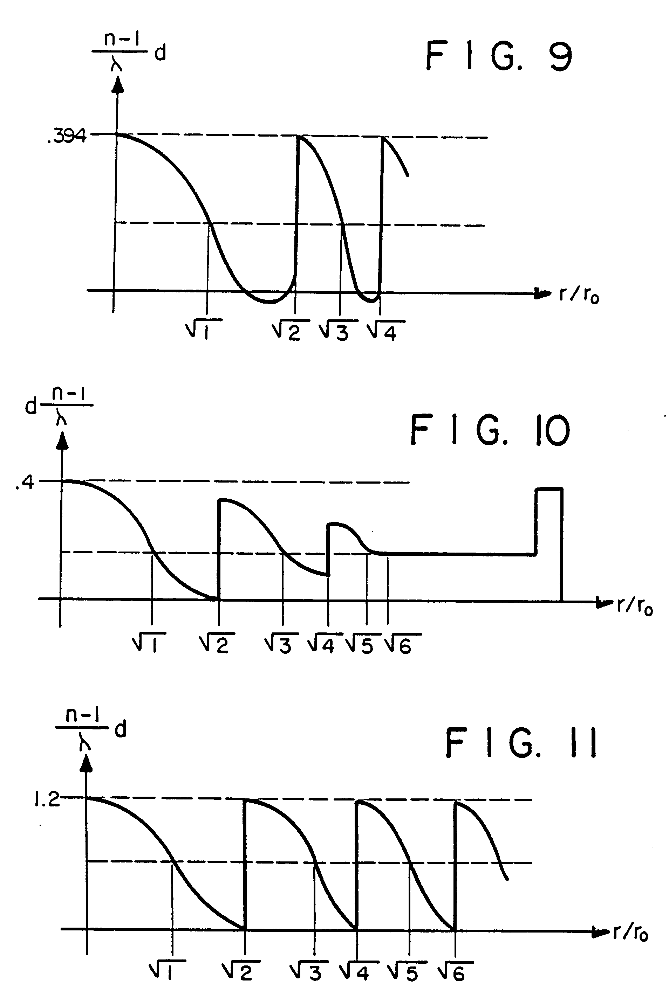

Figure 9 shows a faceted profile where the inclination of the even zones have a bottom curvature which presents two opposite-facing curves before connecting with the non-refractive edge of the step. this embodiment of the invention is characterized by

$$y = 0.394\lambda/(n - 1)\{0.287 + 0.731J_o(4.20 \cdot r^2/2r_o^2)\}$$

$$I_o = I_1 = 0.402$$

wherein $J_o$ is a bessel function.

Figure 10 shows another facet profile that incorporates two phase zone plates and a pure refractive portion. In this embodiment, there is a decrease in depth from full-period zone to full-period zone, though it is not necessary for the decrease to exist throughout the optic zone. For example, the first half of the full-period zones of the optic zone may be only one depth, and the second half of the full-period zones may be progressively reduced in depth. In the preferred modes of such embodiments, each of the steps, whether of the same or different depth, is equally divided along a common plane of the optic zone. The pure refractive portion is preferably in the form of one or more channels which may be incorporated within the optic zone and/or circumscribing the optic zone. The particular embodiment of Figure 10 is characterized by

$$y = \alpha\lambda/(n - 1)\{1/2 + 1/2\cos\pi/2 \cdot r^2/r_o^2\}$$

where $\alpha$ decreases from zone to zone

Figure 11 illustrates a phase zone plate design in which the depth of the echelettes is greater than $\lambda$, in this case $1.2\lambda$. The shape of the profile is a cosine configuration. This embodiment of the configuration of Figure 11 is characterized by

$$y = 1.2\lambda/(n - 1)\{1/2 + 1/2 \cos \pi/2 \cdot r^2/r_o^2\}$$

$$I_o = 0.404 \quad I_1 = 0.358$$

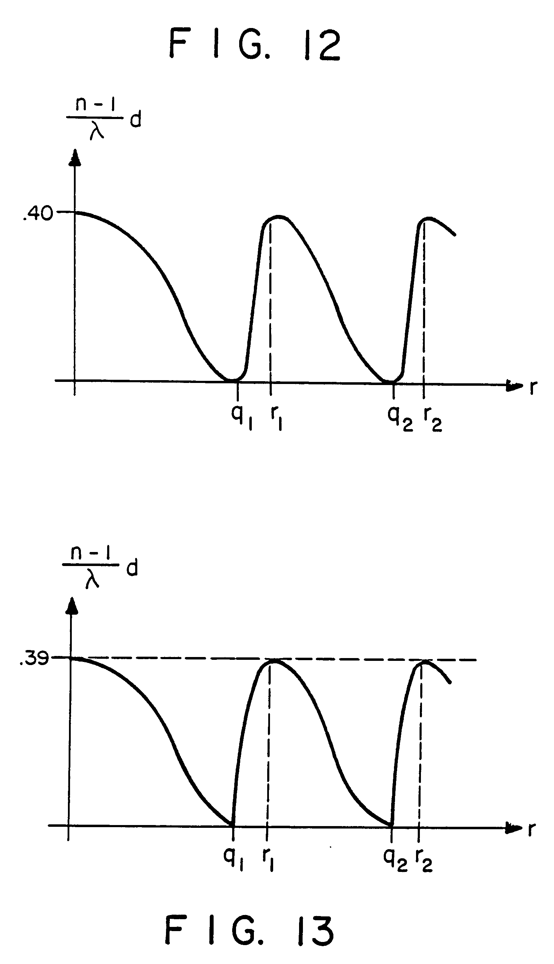

The faceted profiles of Figures 12 and 13 introduce a space reduction in the full period zone at $q_1$ which allows the step to proceed from $q_1$ to $r_2$, etc. This space reduction in the alternating zone is not regarded to alter the $\sqrt{n}$ spacing of the zones. Figure 12 is characterized by

$$y_n = .40\{1/2 + 1/2 \cos[\pi(r^2 - r_{n-1}^2)/(q_n^2 - r_n^2)]\}$$

where $r_{n-1} < r < q_n$

$$y_n = .40\{1/2 + 1/2 \sin[\pi(r^2 - (r_n^2 + q_n^2)/2)/(r_n^2 - q_n^2)]\}$$

where $q_n < r < r_{n-1}$

$$r_n \simeq \sqrt{2\,n\,d\,\lambda}$$
$$q_n = r_{n-1} + (r_n - r_{n-1})/\alpha$$
$$\alpha = 1.086$$

Figure 13 is characterized

$$y_n = .39\{1/2 + 1/2 \cos[\pi(r^2 - r_{n-1}^2)/(q_n^2 - r_n^2)]\}$$

where $r_{n-1} < r < q_n$

$$y_n = .39\{1 - (r - r_n)/(r_n - q_n)^2\}$$

where $q_n < r < r_n$

$$r_n \simeq \sqrt{2\,n\,d\,\lambda}$$
$$q_n = r_{n-1} + (r_n - r_{n-1})/\alpha$$
$$\alpha = 1.086$$

It should be appreciated that the invention is not limited to the exact details of construction shown and described herein for many obvious modifications will occur to persons skilled in the art. The variety of curved profile shapes of the facets is not limited to those specifically illustrated. It should be apparent that many other curved profiles are suitably employable to manufacture optical elements possessing effective splits of the light intensities to the zeroth and first orders to serve as useful bifocal optical elements.

## Claims

1. A method of manufacturing a multifocal diffractive optical element including at least one zone plate containing annular concentric zones which are spaced substantially proportional to $\sqrt{n}$, n being an integer, said zones including a facet with a non-parabolic profile having a depth $D_o$, the method comprising steps of:

   firstly determining a profile depth $D_o$ by:
   - selecting a non-parabolic profile;
   - selecting a design wavelength and zeroth and first order focal lengths;
   - calculating intensities of light at said design wavelength at the zeroth and first order focal points as a function of profile depth, said intensities being determined by evaluating the Kirchhoff diffraction integral for said non-parabolic profile;

   selecting that profile depth to be depth $D_o$ at which the intensities of light are equal at the zeroth and first order focal points; and

   subsequently producing a multifocal optical element having the profile depth $D_o$ thus determined.

2. A method according to claim 1 wherein the facets have curved profiles defined by $d = D_o \times \{\frac{1}{2} + \frac{1}{2} \times \cos(\pi \times r^2/b^2)\}$, where d is the depth of the profile, $D_o$ is the facet depth for the design wavelength, r is the outside radius of the zone, and b is the outside radius of the first zone.

3. A method according to claim 1 wherein the facets have curved profiles substantially corresponding to $d = D_o \{0.287 + 0.73) \times J_o [(4.2 \times r^2)/(2 \times r_o^2)]$ where d is the depth of the profile, $D_o$ is the facet depth for the design wavelength, $J_o$ is a Bessel function, r is the outside radius of the zone, and $r_o$ is the outside radius of the first zone.

4. A method according to any one of the preceding claims wherein the zone plate has a facet profile that varies smoothly without discontinuities.

5. A method according to any one of the preceding claims wherein the zone plate has stepped facets, each stepped facet having a rounded outer corner.

6. A method accordingly to claim 5 wherein each stepped facet has a rounded inner corner.

7. A method according to any one of the preceding claims wherein the design wavelength is within the visible spectrum.

8. A method according to any one of the preceding claims wherein the optical element is an ophthalmic lens.

9. A method according to claim 8 wherein the lens is an intraocular lens.

EP 0 355 230 B1

10. A method according to claim 8 wherein the lens is a contact lens.


## Patentansprüche

1. Verfahren zum Herstellen eines mehrfokalen optischen Beugungselementes, mit zumindest einer Zonenplatte, die ringförmige, konzentrische Zonen enthält, die im wesentlichen proportional zu $\sqrt{n}$ beabstandet sind, wobei n eine ganze Zahl ist, die Zonen zumindest eine Facette mit einem nichtparabolischen Profil einer Tiefe $D_o$ einschließen, und das Verfahren die Schritte aufweist:
zunächst Bestimmen einer Profiltiefe $D_o$ durch:
   - Auswahl eines nichtparabolischen Profils;
   - Auswahl einer Auslegungswellenlänge und Brennweiten nullter und erster Ordnung,
Berechnen der Lichtintensitäten bei der Auslegungswellenlänge an den Brennpunkten nullter und erster Ordnung als Funktion der Profiltiefe, wobei die Intensitäten bestimmt werden durch Auswerten des Kirchhoffschen Brechungs- bzw. Beugungsintegrals für das nichtparabolische Profil,
Auswahl derjenigen Profiltiefe als die Tiefe $D_o$, bei welcher die Intensitäten des Lichtes bei den Brennpunkten nullter und erster Ordnung gleich sind, und anschließendes Herstellen eines optischen Elementes mit mehreren Brennpunkten, welches die so bestimmte Profiltiefe $D_o$ hat.

2. Verfahren nach Anspruch 1, wobei die Facetten einen gekrümmten Profilverlauf haben, der definiert wird durch $d = D_o \times (\frac{1}{2} + \frac{1}{2} \times \cos(\pi \times r^2/b^2))$, wobei d die Tiefe des Profiles, $D_o$ die Facettentiefe für die Auslegungswellenlänge, r der äußere Zonenradius und b der äußere Radius der ersten Zone ist.

3. Verfahren nach Anspruch 1, wobei die Facetten einen gekrümmten Profilverlauf haben, der in etwa entspricht: $d = D_o (0,287 + 0,73) \times J_o [(4,2 \times r^2)/(2 \times r_o)]$, wobei d die Profiltiefe, $D_o$ die Facettentiefe für die Auslegungswellenlänge, $J_o$ eine Besselfunktion, r der Außenradius der Zone und $r_o$ der Außenradius der ersten Zone ist.

4. Verfahren nach einem der vorstehenden Ansprüche, wobei die Zonenplatte ein Facettenprofil hat, welches ohne Diskontinuitäten glatt bzw. gleichmäßig variiert.

5. Verfahren nach einem der vorstehenden Ansprüche, wobei die Zonenplatte abgestufte Facetten hat, wobei jede abgestufte Facette eine abgerundete äußere Ecke hat.

6. Verfahren nach Anspruch 5, wobei jede abgestufte Facette eine abgerundete innere Ecke hat.

7. Verfahren nach einem der vorstehenden Ansprüche, wobei die Auslegungswellenlänge innerhalb des sichtbaren Spektrums liegt.

8. Verfahren nach einem der vorstehenden Ansprüche, wobei das optische Element eine für Augen vorgesehene Linse ist.

9. Verfahren nach Anspruch 8, wobei die Linse eine intraokulare Linse ist.

10. Verfahren nach Anspruch 8, wobei die Linse eine Kontaktlinse ist.


## Revendications

1. Procédé de fabrication d'un élément optique diffractif multifocal incluant au moins une lamelle zonée contenant des zones concentriques annulaires qui sont espacées sensiblement de façon proportionnelle à $\sqrt{n}$, n étant un nombre entier, lesdites zones incluant une facette avec un profil non parabolique ayant une profondeur de $D_o$, le procédé comprenant les étapes :
   en premier, de détermination d'une profondeur de profil $D_o$ en :
   - sélectionnant un profil non parabolique ;
   - sélectionnant une longueur d'onde théorique et des distances focales des zéroième et premier ordres ;
   - calculant des intensités de lumière à ladite longueur d'onde théorique, au droit des points focaux des zéroième et premier ordres en fonction de la profondeur de profil, lesdites intensités étant déterminées par la valorisation de l'intégrale de diffraction de Kirchhoff pour ledit profil non parabolique ;

- sélectionnant cette profondeur de profil comme profondeur $D_o$ à laquelle les intensités de lumière sont égales au droit des points focaux des zéroième et premier ordres ; et

ensuite, de production d'un élément optique multifocal ayant la profondeur de profil $D_o$ ainsi déterminée.

2. Procédé selon la revendication 1, dans lequel les facettes ont des profils incurvés définis par $d = D_o \times \{1/2 + 1/2 \times \cos(\pi \times r^2 / b^2)\}$, où d est la profondeur de profil, $D_o$ est la profondeur de facette pour la longueur d'onde théorique, r est le rayon extérieur de la zone, et b est le rayon extérieur de la première zone.

3. Procédé selon la revendication 1, dans lequel les facettes ont des profils incurvés correspondant sensiblement à $d = D_o \times (0,287 + 0,73) \times J_o [(4,2 \times r^2) / (2 \times r_o^2)]$, où d est la profondeur de profil, $D_o$ est la profondeur de facette pour la longueur d'onde théorique, $J_o$ est une fonction de Bessel, r est le rayon extérieur de la zone, et $r_o$ est le rayon extérieur de la première zone.

4. Procédé selon l'une quelconque des revendications précédentes, dans lequel la lamelle zonée a un profil de facettes qui varie régulièrement sans discontinuités.

5. Procédé selon l'une quelconque des revendications précédentes, dans lequel la lamelle zonée a des facettes étagées, chaque facette étagée ayant un coin extérieur arrondi.

6. Procédé selon la revendication 5, dans lequel chaque facette étagée ayant un coin intérieur arrondi.

7. Procédé selon l'une quelconque des revendications précédentes, dans lequel la longueur d'onde théorique est à l'intérieur du spectre visible.

8. Procédé selon l'une quelconque des revendications précédentes, dans lequel l'élément optique est une lentille ophtalmique.

9. Procédé selon la revendication 8, dans lequel la lentille est une lentille intraoculaire.

10. Procédé selon la revendication 8, dans lequel la lentille est une lentille de contact.

F I G. 3

F I G. 1
(PRIOR ART)

EP 0 355 230 B1

F I G. 2
(PRIOR ART)

F I G. 4

F I G. 5

# F I G. 2a

# F I G. 4a

$\dfrac{n-1}{\lambda} d$

F I G. 6

.405

$\sqrt{1}$  $\sqrt{2}$  $\sqrt{3}$  $\sqrt{4}$  $\sqrt{5}$

$r/r_o$

$\dfrac{n-1}{\lambda} d$

F I G. 7

.31

$\sqrt{1}$  $\sqrt{2}$  $\sqrt{3}$  $\sqrt{4}$  $\sqrt{5}$

$r/r_o$

$\dfrac{n-1}{\lambda} d$

F I G. 8

.21

$r/r_o$

-.21

$\sqrt{1}$  $\sqrt{2}$  $\sqrt{3}$  $\sqrt{4}$

$$\frac{n-1}{\lambda}d$$

FIG. 9

.394

$\sqrt{1}$ $\sqrt{2}$ $\sqrt{3}$ $\sqrt{4}$

$r/r_0$

$$d\frac{n-1}{\lambda}$$

FIG. 10

.4

$\sqrt{1}$ $\sqrt{2}$ $\sqrt{3}$ $\sqrt{4}$ $\sqrt{5}$ $\sqrt{6}$

$r/r_0$

$$\frac{n-1}{\lambda}d$$

FIG. 11

1.2

$\sqrt{1}$ $\sqrt{2}$ $\sqrt{3}$ $\sqrt{4}$ $\sqrt{5}$ $\sqrt{6}$

$r/r_0$

18

# F I G. 12

# F I G. 13